# EUROPÄISCHE PATENTSCHRIFT

(11) **EP 3 517 035 B1**
(45) Veröffentlichungstag und Bekanntmachung des Hinweises auf die Patenterteilung: **24.03.2021**
(21) Anmeldenummer: 18154188.9
(22) Anmeldetag: 30.01.2018
(51) Int. Cl.: A61B 6/00, A61B 17/34

(54) **INTEGRIERBARE BIOPSIEEINHEIT UND MAMMOGRAPHIEEINRICHTUNG**
INTEGRABLE BIOPSY UNIT AND MAMMOGRAPHY DEVICE
UNITÉ DE BIOPSIE INTÉGRABLE ET DISPOSITIF DE MAMMOGRAPHIE

(43) Veröffentlichungstag der Anmeldung: 31.07.2019
(73) Patentinhaber: Siemens Healthcare GmbH, 91052 Erlangen (DE)
(72) Erfinder: Bechtold, Mario, 91334 Hemhofen (DE); Gollwitzer, Helmut, 92681 Erbendorf (DE); Neuber, Wolfgang, 92690 Pressath (DE); Ritter, Juliane, 91054 Erlangen (DE)

(56) Entgegenhaltungen:
- WO-A1-2012/032810
- DE-A1-102006 004 590
- DE-A1-102011 081 420

## Beschreibung

Die Erfindung betrifft eine Mammographieeinrichtung mit darin integrierter Biopsieeinheit.

Zur Durchführung von Biopsien bei einer Mammographie sind Mammographieeinrichtungen bekannt, auf die eine Biopsieeinheit aufgesetzt werden kann. Eine solche Biopsieeinheit umfasst insbesondere eine Aufnahme für eine Biopsienadel, ein Mittel zum Verschieben der Aufnahme zwischen einer ersten und zumindest einer vorbestimmten zweiten Position und eine Steuereinheit zum Steuern des Verschiebemittels und zum Vorbestimmen der zweiten Position.

Bekannte Mammographieeinrichtungen umfassen einen Hauptkörper, der sich z. B. in einer vertikalen Richtung erstreckt, eine Bilderfassungseinrichtung und eine Kompressionseinheit. Bilderfassungseinrichtung und/oder Kompressionseinheit sind mit diesem Hauptkörper starr oder schwenkbar verbunden. Dazu können Bilderfassungseinrichtung und Kompressionseinheit auf einem mit dem Hauptkörper verbundenen schwenkbaren Bogen angeordnet sein. Für solche Mammographieeinrichtungen sind Biopsieeinheiten bekannt, die zum Durchführen einer Biopsie mit der Mammographieeinrichtung verbunden werden.

In einer bekannten Einrichtung kann die Biopsieeinheit auf einem Auflagetisch der Kompressionseinheit aufgesetzt werden. Für jede Verwendung der Biopsieeinheit muss die Biopsieeinheit neu justiert werden, damit die Biopsie an der dafür vorgesehenen Stelle durchgeführt werden kann. Überdies ist es für die Verwendung einer solchen Biopsieeinheit notwendig, dass ein Kompressionselement der Kompressionseinheit mit einem speziellen Rahmen versehen ist, der die Biopsieeinheit umgeben kann.

Eine weitere bekannte Biopsieeinheit ist als Aufsatz für eine weitere Mammographieeinrichtung ausgeführt. Die Biopsieeinheit wird in zwei vertikalen Führungsnuten des Hauptkörpers der Mammographieeinrichtung aufgenommen. Auch bei dieser bekannten Biopsieeinheit ist es notwendig, die Biopsieeinheit vor jeder Verwendung neu zu justieren.

Aus der WO 2012/032810 A1 ist eine Mammographieeinrichtung bekannt, welche es ermöglicht, Biopsien an Gewebe-Attrappen durchzuführen. Die Vorrichtung umfasst dabei eine Platte, welche als Auflagefläche für die Attrappe dient, eine Kompressionsplatte und eine Röntgenquelle sowie einen Detektor. Eine Biopsieeinheit ist mit Hilfe eines Schwenkarms, welcher aus zwei horizontal beweglichen Armen besteht, auf der Kompressionsplatte angebracht. Mit der Biopsieeinheit können aus Gewebeattrappen Proben entnommen werden.

Aus der DE 10 2011 081 420 A1 ist ein Mammographiegerät mit einer Biopsieeinheit bekannt, wobei die Biospieeinheit einen mehrgliedrigen Arm mit mindestens zwei Gelenken umfasst. In einer Ausführungsform kann dabei die Biopsieeinheit eine Robotersteuerung aufweisen, mit welcher der mehrgliedrige Arm und die Biopsienadel gesteuert werden kann. Die Biopsieeinheit ist dabei beweglich mit dem Mammographiegerät verbunden. Hiermit wird ermöglicht, dass die Biopsieeinheit in eine Arbeitsposition, eine vorprogrammierte Position oder in eine Parkposition gebracht werden kann.

Aus der DE 10 2006 004 590 A1 ist eine Mammographieeinrichtung mit einem Stativ, einer Geräteträgereinheit und einem Objekttisch bekannt. Die Bestrahlungseinheit ist dabei an einem C-bogenförmigen Tragarm befestigt, welcher in einer zu einer Horizontalachse senkrechten Schwenkachse liegt. Der Tragarm kann dabei wie ein verfahrbarer Teleskoparm ausgebildet sein. Ebenfalls kann die Mammographieeinheit eine Biopsieeinheit aufweisen, welche an der Kompressionseinheit schwenkbar gelagert ist und zwischen einer Parkposition, welche in einem vom C-bogenförmigen Tragarm umspannten freien Parkraum angeordnet ist, und einer Biopsieposition verfahrbar ist.

Die Aufgabe der Erfindung ist es, die Nachteile nach dem Stand der Technik zu beseitigen. Insbesondere soll eine Biopsieeinheit und eine Mammographieeinrichtung angegeben werden, die einen erhöhten Bedienkomfort zum Durchführen einer Biopsie aufweist.

Diese Aufgabe wird hinsichtlich der Mammographieeinrichtung durch die Merkmale des Anspruchs 1 gelöst. Vorteilhafte Ausgestaltungen der Erfindung sind Gegenstand der abhängigen Ansprüche.

Die Biopsieeinheit zur Befestigung an einer Mammographieeinrichtung, umfasst
- eine Befestigungseinrichtung,
- eine Aufnahme für eine Biopsienadel,
wobei die Biopsieeinheit fest mit der Mammographieeinrichtung verbindbar ist, und zwischen einem Ruhezustand und einem Betriebszustand verschwenkbar ist.

Im Betriebszustand ist die Biopsieeinheit so ausgerichtet, dass mit der Biopsieeinheit eine Biopsie durchgeführt werden kann. Im Ruhezustand kann mit der Biopsieeinheit keine Biopsie durchgeführt werden.

In einer Ausgestaltung umfasst die Biopsieeinheit einen ersten Arm, der an der Befestigungseinrichtung um eine erste Achse beweglich ist, und einen zweiten Arm, der um eine zweite Achse beweglich ist.

Die erste Achse kann eine horizontale Achse sein und/oder die zweite Achse kann parallel zum ersten Arm verlaufen.

In einer Ausgestaltung ist der zweite Arm auf der vom ersten Arm abgewandten Seite mit einem dritten Arm verbunden, der insbesondere einen Winkel von 90° bis 100° zum zweiten Arm aufweist und/oder um eine parallel zum zweiten Arm verlaufende dritte Achse beweglich ist. Der Winkel zwischen zweiten und dritten Arm kann fest in dem Winkelbereich gewählt sein oder einstellbar sein.

Der erste Arm, der zweite Arm und/oder dritte Arm können längenverstellbar ausgestaltet sein. Damit wird die Flexibilität der Biopsieeinrichtung erhöht.

Zweckmäßigerweise verläuft der dritte Arm in einer Betriebsposition im Wesentlichen parallel zum ersten Arm.

In einer Ausgestaltung ist die Aufnahme für eine Biopsienadel am dritten Arm angeordnet.

Die erfindungsgemäße Mammographieeinrichtung umfasst neben der Biopsieeinheit
- einen Hauptkörper, der sich insbesondere in einer vertikalen Richtung erstreckt,
- eine Bilderfassungseinrichtung,
- eine Kompressionseinheit und ein Gehäuse mit einer Öffnung , das so ausgestaltet ist, dass die Biopsieeinheit im Ruhezustand vollständig in dem Gehäuse aufgenommen ist und dass die Biopsieeinheit im Betriebszustand beim Verschwenken vom Ruhezustand in den Betriebszustand zumindest teilweise aus dem Gehäuse herausgeschwenkt werden kann.

Die Biopsieeinheit kann so bei Bedarf einfach ausgeklappt werden und muss nicht vor jeder Benutzung zunächst mit der Mammographieeinrichtung verbunden und nachfolgend justiert werden. Bei der Mammographieeinrichtung bleibt eine Justage auch über das Verschwenken in den Ruhezustand erhalten.

Zweckmäßigerweise weist die Mammographieeinrichtung ein Gehäuse auf, das so ausgestaltet ist, dass die Biopsieeinheit im Ruhezustand in dem Gehäuse aufgenommen ist. Das Gehäuse kann insbesondere ein ringförmiges Gehäuse sein. Zweckmäßigerweise ist auf einer äußeren Ringfläche zumindest eine Öffnung vorgesehen, so dass die Biopsieeinheit beim Verschwenken vom Ruhezustand in den Betriebszustand teilweise aus dem Gehäuse herausgeschwenkt werden kann. Die Öffnung kann auf der äußeren Ringfläche verschiebbar sein.

In einer weiteren Ausgestaltung weist die Einrichtung eine einzige Steuereinrichtung zur Steuerung der Bilderfassungeinrichtung und zur Steuerung der Biopsieeinheit auf oder weist miteinander kommunizierende Steuereinrichtungen zur Steuerung der Bilderfassungseinrichtung und zur Steuerung der Biopsieeinheit auf. Durch das Vorsehen einer gemeinsamen Steuereinrichtung oder miteinander kommunizierender Steuereinrichtungen wird die Benutzung der Mammographieeinrichtung mit der Biopsieeinheit erleichtert.

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung der Ausführungsbeispiele, die im Zusammenhang mit den Zeichnungen näher erläutert werden.

Für eine weitere Beschreibung der Erfindung wird auf die Ausführungsbeispiele der Zeichnungen verwiesen. Es zeigt in einer schematischen Prinzipskizze:
- Fig. 1: eine Biopsieeinheit im Ruhezustand
- Fig. 2: eine Biopsieeinheit im Betriebszustand
- Fig. 3: eine erste Mammographieeinrichtung mit Biopsieeinheit im Ruhezustand
- Fig. 4: eine erste Mammographieeinrichtung mit Biopsieeinheit im Betriebszustand
- Fig. 5: die erfindungsgemäße Mammographieeinrichtung mit Biopsieeinheit im Ruhezustand
- Fig. 6: die erfindungsgemäße Mammographieeinrichtung mit Biopsieeinheit im Betriebszustand

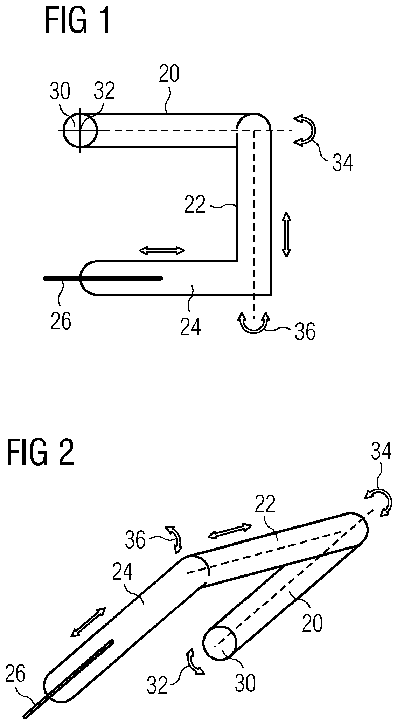

Fig. 1 zeigt die Biopsieeinheit 10 in einem Ruhezustand. Ein erster Arm 20 kann mit an der Befestigungseinrichtung 30 um eine horizontale erste Achse 32 gedreht werden. Der erste Arm 20 ist im Beispiel längenverstellbar ausgestaltet. Vom ersten Arm 20 erstreckt sich ein zweiter Arm 22, der ebenfalls längenverstellbar ausgestaltet ist und vom zweiten Arm 22 erstreckt sich ein dritter Arm 24, der auch längenverstellbar ist. Der zweite Arm 22 lässt sich um eine zweite Achse 34 bewegen, die parallel zum ersten Arm 20 ausgerichtet ist. Der dritte Arm 24 lässt sich um eine dritte Achse 36 bewegen, die parallel zum zweiten Arm 22 verläuft. Der dritte Arm 24 dient zur Aufnahme einer Biopsienadel 26.

Im in Fig. 2 gezeigten Betriebszustand ist der zweite Arm 22 um die sich parallel zum ersten Arm 20 erstreckende Achse verschwenkt. Durch Drehen der Biopsieeinheit 10 um die erste Achse 32 und die parallel zum ersten Arm 20 verlaufende zweite Achse 34 und die dritte Achse 36 kann mit der Biopsieeinheit 10 eine Biopsie an der gewünschten Stelle des Objekts durchgeführt werden.

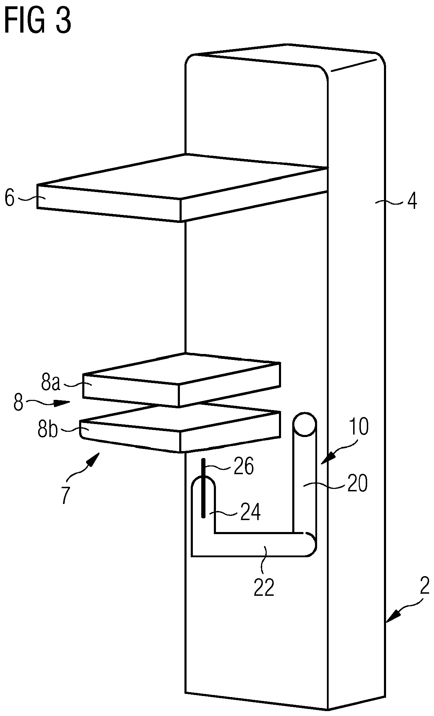

Fig. 3 zeigt eine erste Ausgestaltung einer Mammographieeinrichtung 2. Die Mammographieeinrichtung 2 umfasst einen Hauptkörper 4, der sich in einer vertikalen Richtung erstreckt. Ein Hauptkörper 4 kann sich in einer anderen Ausgestaltung auch horizontal erstrecken. Im oberen Bereich des Hauptköpers 4 ist an einem Ausleger eine Röntgenquelle 6 angeordnet, die mit dem Detektor die Bilderfassungseinrichtung bildet. An dem Hauptkörper 4 ist die Biopsieeinrichtung 10 befestigt, die sich im Ruhezustand befindet.

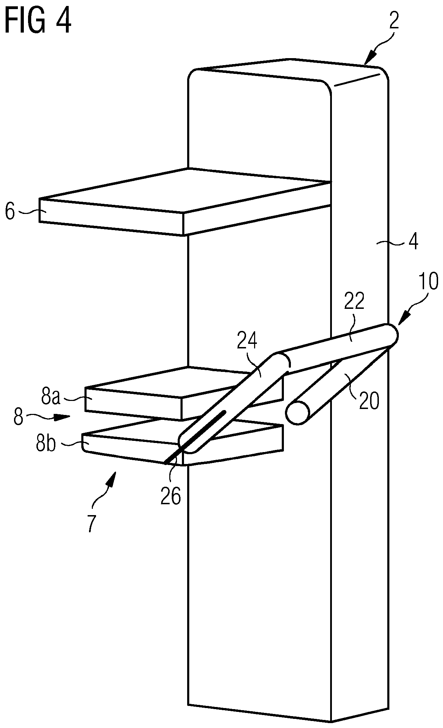

Fig. 4 zeigt die in Fig. 3 gezeigte Ausgestaltung der Mammographieeinrichtung mit der Biopsieeinheit 10 im Betriebszustand. Die Biopsieeinheit 10 ist vom Hauptkörper 4 weg geschwenkt, so dass sich der zweite Arm 22 senkrecht zur Erstreckungsrichtung des Hauptkörpers 4 erstreckt. Ein dritter Arm 24 erstreckt sich im Wesentlichen senkrecht zum zweiten Arm 22 und dient zur Aufnahme einer Biopsienadel 26.

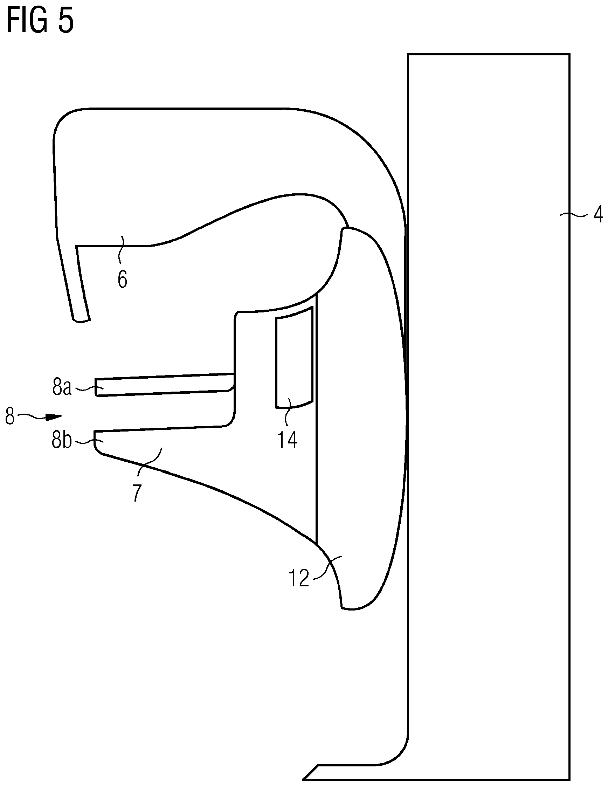

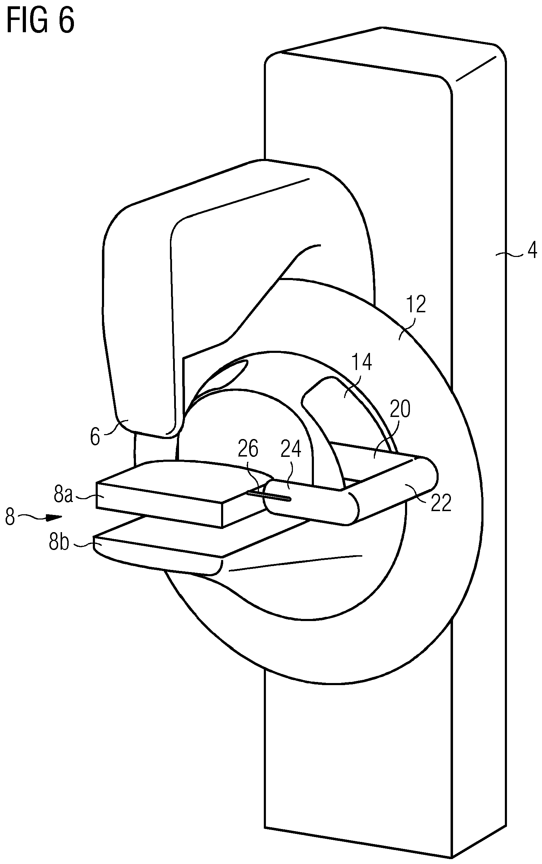

Fig. 5 und 6 zeigen die erfindungsgemäße Mammographieeinrichtung 2. Diese weist zusätzlich ein Gehäuse 12 mit zumindest einer Öffnung 14 auf. Das Gehäuse 12 ist mit dem Hauptkörper 4 verbunden. Auf der dem Hauptkörper 4 abgewandten Seite des Gehäuses 12 ist die Kompressionseinheit aus Kompressionselement 8a und Objekttisch 8b so angeordnet, dass mit der Röntgenquelle 6 ein in der Kompressionseinheit 8 befindliches Objekt durchleuchtet werden kann. Die Strahlung wird mit einem im Bereich des Objekttisches 8b befindlichen Detektors 7 aufgenommen. In Fig. 5 ist die Biopsieeinheit 10 im Ruhezustand und vollständig im Gehäuse 12 aufgenommen. In Fig. 6 ist die Biopsieeinheit 10 im Betriebszustand und springt aus einer Öffnung 14 des Gehäuses teilweise hervor. Die Öffnung 14 kann größer als gezeigt sein, so dass sie z.B. eine Schwenkbewegung von ±90° um die erste Achse 32 erlaubt, oder im Gehäuse 12 verschiebbar sein, um eine entsprechende Schwenkbewegung zu erlauben. Der erste Arm 20 ist teilweise außerhalb des Gehäuses 12 während der zweite Arm 22 und der dritte Arm 24 sich vollständig außerhalb des Gehäuses 12 befindet.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Mammographieeinrichtung (2), umfassend:
- einen Hauptkörper (4), der sich in einer vertikalen Richtung erstreckt,
- eine Bilderfassungseinrichtung (6,7),
- eine Kompressionseinheit (8) und
- eine Biopsieeinheit (10), umfassend
- eine Befestigungseinrichtung (30),
- eine Aufnahme für eine Biopsienadel (26),
wobei die Biopsieeinheit (10) fest mit der Mammographieeinrichtung (2) verbunden ist, und zwischen einem Ruhezustand und einem Betriebszustand verschwenkbar ist,
**dadurch gekennzeichnet, dass**
die Mammographieeinrichtung (2) ein Gehäuse (12) mit einer Öffnung (14) aufweist, das so ausgestaltet ist, dass die Biopsieeinheit (10) im Ruhezustand vollständig in dem Gehäuse (12) aufgenommen ist und dass die Biopsieeinheit (10) beim Verschwenken vom Ruhezustand in den Betriebszustand zumindest teilweise aus dem Gehäuse herausgeschwenkt werden kann.

2. Mammographieeinrichtung (2) nach Anspruch 1, **dadurch gekennzeichnet, dass** die Mammographieeinrichtung (2) ein ringförmiges Gehäuse (12) aufweist, wobei auf deren äußeren Ringfläche zumindest eine Öffnung (14) vorgesehen ist, sodass die Biopsieeinheit (10) beim Verschwenken vom Ruhezustand in den Betriebszustand teilweise aus dem Gehäuse (12) herausgeschwenkt werden kann.

3. Mammographieeinrichtung (2) mit einer Biopsieeinrichtung (10) nach einem der vorhergehenden Ansprüche, **dadurch gekennzeichnet, dass** die Biopsieeinrichtung (10) einen ersten Arm (20) umfasst, der an der Befestigungseinrichtung (30) um eine erste Achse (32) beweglich ist, und einen zweiten Arm (22) umfasst, der um eine zweite Achse (34) beweglich ist.

4. Mammographieeinrichtung (2) mit einer Biopsieeinrichtung (10) nach Anspruch 3, **dadurch gekennzeichnet, dass** die erste Achse (32) eine horizontale Achse ist und/oder die zweite Achse (34) parallel zum ersten Arm (20) verläuft.

5. Mammographieeinrichtung (2) mit einer Biopsieeinrichtung (10) nach einem der Ansprüche 3-4, **dadurch gekennzeichnet, dass** der zweite Arm (22) auf der vom ersten Arm (20) abgewandten Seite mit einem dritten Arm (24) verbunden ist, der insbesondere einen Winkel von 90° bis 100° zum zweiten Arm (22) aufweist und/oder um eine parallel zum zweiten Arm verlaufende dritte Achse (36) beweglich ist.

6. Mammographieeinrichtung (2) mit einer Biopsieeinrichtung (10) nach einem der Ansprüche 3-5, **dadurch gekennzeichnet, dass** der erste Arm (20), der zweite Arm (22) und/oder dritte Arm (24) längenverstellbar ist.

7. Mammographieeinrichtung (2) mit einer Biopsieeinrichtung (10) nach Anspruch 5 oder 6, **dadurch gekennzeichnet, dass** der dritte Arm (24) in einer Betriebsposition sich im Wesentlichen senkrecht zum zweiten Arm (22) erstreckt

8. Mammographieeinrichtung (2) nach einem der Ansprüche 5-7, **dadurch gekennzeichnet, dass** im Betriebszustand der erste Arm (20) teilweise außerhalb des Gehäuses (12) ist, während der zweite (22) und dritte Arm (24) sich vollständig außerhalb des Gehäuses (12) befinden.

9. Mammographieeinrichtung (2) mit einer Biopsieeinrichtung (10) nach einem der Ansprüche 5 bis 8, **dadurch gekennzeichnet, dass** die Aufnahme für eine Biopsienadel (26) am dritten Arm (24) angeordnet ist.

10. Mammographieeinrichtung (2) nach einem der vorhergehenden Ansprüche, **dadurch gekennzeichnet, dass** die Einrichtung eine einzige Steuereinrichtung zur Steuerung der Bilderfassungseinrichtung und zur Steuerung der Biopsieeinheit (10) aufweist oder miteinander kommunizierende Steuereinrichtungen zur Steuerung der Bilderfassungseinrichtung und zur Steuerung der Biopsieeinheit (10) aufweist.

## Claims

1. Mammography device (2), comprising:
- a main body (4) which extends in a vertical direction,
- an image acquisition device (6, 7),
- a compression unit (8), and
- a biopsy unit (10) comprising
- a fixing device (30),
- a receptacle for a biopsy needle (26),
wherein the biopsy unit (10) is permanently connected to the mammography device (2), and can be pivoted between a rest state and an operating state,
**characterised in that**
the mammography device (2) has a housing (12) with an opening (14) which is configured such that the biopsy unit (10) is completely accommodated in the housing (12) in the rest state and that the biopsy unit (10) can be at least partially pivoted out of the housing when pivoted from the rest state to the operating state.

2. Mammography device (2) according to claim 1, **characterised in that** the mammography device (2) has an annular housing (12), wherein at least one opening (14) is provided on an outer ring surface, such that the biopsy unit (10) can be partially pivoted out of the housing (12) when pivoted from the rest state to the operating state.

3. Mammography device (2) with a biopsy unit (10) according to one of the preceding claims, **characterised in that** the biopsy unit (10) comprises a first arm (20) which on the fixing device (30) can move about a first axis (32), and a second arm (22) which can move about a second axis (34).

4. Mammography device (2) with a biopsy unit (10) according to claim 3, **characterised in that** the first axis (32) is a horizontal axis and/or the second axis (34) runs parallel to the first arm (20).

5. Mammography device (2) with a biopsy unit (10) according to one of claims 3-4, **characterised in that** the second arm (22) is connected to a third arm (24) on the side facing away from the first arm (20), said third arm (24) being in particular at an angle of between 90° and 100° to the second arm (22) and/or being able to be moved about a third axis (36) running parallel to the second arm.

6. Mammography device (2) with a biopsy unit (10) according to one of claims 3-5, **characterised in that** the first arm (20), the second arm (22) and/or the third arm (24) can be adjusted in terms of length.

7. Mammography device (2) with a biopsy unit (10) according to claim 5 or 6, **characterised in that** the third arm (24) extends substantially perpendicular to the second arm (22) in an operating position.

8. Mammography device (2) according to one of claims 5-7, **characterised in that** in the operating state the first arm (20) is partially outside the housing (12), while the second arm (22) and the third arm (24) are completely outside the housing (12).

9. Mammography device (2) with a biopsy unit (10) according to one of claims 5 to 8, **characterised in that** the receptacle for a biopsy needle (26) is arranged on the third arm (24).

10. Mammography device (2) according to one of the preceding claims, **characterised in that** the device has a single control device for controlling the image acquisition device and for controlling the biopsy unit (10) or has intercommunicating control devices for controlling the image acquisition device and for controlling the biopsy unit (10).

## Revendications

1. Dispositif (2) de mammographie comprenant :
- un corps (4) principal, qui s'étend dans une direction verticale,
- un dispositif (6, 7) de saisie d'images,
- une unité (8) de compression et
- une unité (10) de biopsie, comprenant
- un dispositif (30) de fixation,
- un logement pour une aiguille (26) de biopsie,
dans lequel l'unité (10) de biopsie est reliée fixement au dispositif (2) de mammographie et peut pivoter entre un état de repos et un état de fonctionnement,
**caractérisé en ce que**
le dispositif (2) de mammographie a un boîtier (12) ayant une ouverture (14) qui est conformée de manière à ce que l'unité (10) de biopsie, soit dans l'état de repos, reçue complètement dans le boîtier (12) et de manière à ce que l'unité (10) de biopsie puisse, lors du pivotement de l'état de repos à l'état de fonctionnement, pivoter au moins en partie hors du boîtier.

2. Dispositif (2) de mammographie suivant la revendication 1, **caractérisé en ce que** le dispositif (2) de mammographie a un boîtier (12) en forme d'anneau, dans lequel, sur sa surface annulaire extérieure est prévue au moins une ouverture (14) de manière à ce que l'unité (10) de biopsie puisse, lors du pivotement de l'état de repos à l'état de fonctionnement, pivoter au moins en partie hors du boîtier (12).

3. Dispositif (2) de mammographie comprenant un dispositif (10) de biopsie suivant l'une des revendications précédentes, **caractérisé en ce que** le dispositif (10) de biopsie comprend un premier bras (20), qui est mobile sur le dispositif (30) de fixation autour d'un premier axe (32), et comprend un deuxième bras (22), qui est mobile autour d'un deuxième axe (34).

4. Dispositif (2) de mammographie comprenant un dispositif (10) de biopsie suivant la revendication 3, **caractérisé en ce que** le premier axe (32) est un axe horizontal et/ou le deuxième axe (34) s'étend parallèlement au premier bras (20).

5. Dispositif (2) de mammographie comprenant un dispositif (10) de biopsie suivant l'une des revendications 3 à 4, **caractérisé en ce que** le deuxième bras (22) est relié, du côté loin du premier bras (20) à un troisième bras (24), qui fait notamment un angle de 90° à 100° avec le deuxième bras (22) et/ou qui est mobile autour d'un troisième axe (36) s'étendant parallèlement au deuxième bras.

6. Dispositif (2) de mammographie comprenant un dispositif (10) de biopsie suivant l'une des revendications 3 à 5, **caractérisé en ce que** le premier bras (20) et le deuxième bras (22) et/ou le troisième bras (24) sont réglables en longueur.

7. Dispositif (2) de mammographie comprenant un dispositif (10) de biopsie suivant l'une des revendications 5 ou 6, **caractérisé en ce que** le troisième bras (24) s'étend, dans une position de fonctionnement, sensiblement perpendiculairement au deuxième bras (22).

8. Dispositif (2) de mammographie comprenant un dispositif (10) de biopsie suivant l'une des revendications 5 à 7, **caractérisé en ce que**, dans l'état de fonctionnement, le premier bras (20) est en partie à l'extérieur du boîtier (12), tandis que le deuxième bras (22) et le troisième bras (24) se trouvent complètement à l'extérieur du boîtier (12).

9. Dispositif (2) de mammographie comprenant un dispositif (10) de biopsie suivant l'une des revendications 5 à 8, **caractérisé en ce que** le logement pour un aiguille (26) de biopsie est mis sur le troisième bras (24).

10. Dispositif (2) de mammographie comprenant un dispositif (10) de biopsie suivant l'une des revendications précédentes, **caractérisé en ce que** le dispositif a un dispositif de commande unique pour la commande du dispositif de saisie d'image et pour la commande de l'unité (10) de biopsie ou des dispositifs de commande communiquant entre eux pour la commande du dispositif de saisie d'images et pour la commande de l'unité (10) de biopsie.
